(19) Europäisches Patentamt
European Patent Office
Office européen des brevets

(11) **EP 2 336 762 B1**

(12) **EUROPÄISCHE PATENTSCHRIFT**

(45) Veröffentlichungstag und Bekanntmachung des Hinweises auf die Patenterteilung:
**18.10.2017 Patentblatt 2017/42**

(51) Int Cl.:
*G01N 27/414* (2006.01)   *G01N 33/00* (2006.01)

(21) Anmeldenummer: **10189945.8**

(22) Anmeldetag: **04.11.2010**

(54) **Kohlendioxid-Sensor und zugehöriges Verfahren zur Erzeugung eines Gasmesswerts**

Carbon dioxide sensor and method for generating measured gas values

Capteur de dioxyde de carbone et procédé correspondant pour la production d'une valeur de mesure de gaz

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **14.12.2009 DE 102009058072**

(43) Veröffentlichungstag der Anmeldung:
**22.06.2011 Patentblatt 2011/25**

(73) Patentinhaber: **Siemens Aktiengesellschaft
80333 München (DE)**

(72) Erfinder:
• **Fleischer, Maximilian
85635 Höhenkirchen (DE)**
• **Pohle, Roland
85570 Herdweg (DE)**
• **Stegmeier, Stefan
81543 München (DE)**

(56) Entgegenhaltungen:
**EP-A2- 1 176 418     DE-A1- 3 109 224
DE-A1- 3 526 348     DE-A1-102007 057 520**

• **ENDRES H-E ET AL: "A capacitive CO2 sensor system with suppression of the humidity interference", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, ELSEVIER S.A, CH, Bd. 57, Nr. 1-3, 7. September 1999 (1999-09-07), Seiten 83-87, XP004252989, ISSN: 0925-4005, DOI: 10.1016/S0925-4005(99)00060-X**

EP 2 336 762 B1

**Beschreibung**

Die Erfindung betrifft eine Vorrichtung zur Detektion des Kohlendioxidgehalts von Luft. Weiterhin betrifft die Erfindung ein Verfahren zur Erzeugung eines Gasmesswerts, der die Kohlendioxidkonzentration in Luft repräsentiert.

Die Detektion von Kohlendioxid ist für eine Reihe von Applikationen von hohem Interesse. Beispiele sind die Beurteilung der Luftgüte in Innenräumen, energieeffizientes Ansteuern von Klimaanlagen oder die Kontrolle gereinigter Luft. Ziel der Detektion von Kohlendioxid kann eine Erhöhung des Komforts sein. Es ist aber auch möglich, unter Umständen erhebliche Energieeinsparungen zu erzielen.

So kann beispielsweise bei einem gut isolierten Gebäude nahezu die Hälfte der für eine Klimatisierung benötigten Energie durch eine bedarfsgerechte Klimatisierung eingespart werden. Der Bedarf orientiert sich dabei unter anderem am Kohlendioxid-Gehalt der Luft. Auch im Automobilbereich ist eine bedarfsgerechte Belüftung und Klimatisierung des Fahrgastinnenraums vorteilhaft. Ein Schätzwert für die Reduzierung des Verbrauchs für die Klimatisierung beträgt 0,3 l auf 100 km.

Kohlendioxid tritt bei normalen Umgebungsbedingungen in der Luft in einer Konzentration von ca. 380-400 ppm auf. Ein Sensor für Kohlendioxid muss ausgehend von dieser Basiskonzentration in der Lage sein, erhöhte Konzentrationen bis beispielsweise 4000 ppm zu detektieren. Problematisch ist dabei, dass das Kohlendioxidmolekül ein lineares, symmetrisches Molekül ist und daher kein elektrisches Dipolmoment vorhanden ist, welches bei verschiedenen Transducer-Prinzipien ein Sensorsignal bewirken kann. Weiterhin ist das Molekül chemisch sehr unreaktiv.

Momentan sehr erfolgreiche Methoden zur Konzentrationsbestimmung von Kohlendioxid sind daher vor allem im Bereich der optischen Spektroskopie zu finden. Hierbei wird ausgenutzt, dass Kohlendioxid in bestimmten Wellenlängenbereichen, beispielsweise bei etwa 4,3 $\mu$m Wellenlänge, Licht absorbiert. Hierdurch ist eine genaue und selektive Messung der Konzentration von Kohlendioxid möglich. Dabei kommt es auf die chemische Reaktivität des Kohlendioxids nicht an. Nachteilig an der optischen Spektroskopie sind jedoch der komplexe Aufbau der Messsysteme und der erhebliche Aufwand, der zur Auswertung der gemessenen Spektren erforderlich ist. Das führt letztlich zu verhältnismäßig großen und teuren Messsystemen.

Festkörpersensoren wie beispielsweise Halbleiter-Gassensoren vermeiden die Nachteile der optischen Messsysteme. Sie sind klein, durch Massenproduktion im Vergleich extrem billig herzustellen und benötigen eine weniger komplexe Signalauswertung. Nachteilig bei Festkörpersensoren ist jedoch, dass sie auf eine gewisse Reaktivität der zu messenden Moleküle angewiesen sind und gleichzeitig aber alle Moleküle detektieren, die eben eine gewisse Reaktivität aufweisen.

Anders formuliert haben die Festkörpersensoren eine geringe Selektivität. Das macht vor allem die Messung wenig reaktiver Spezies wie Kohlendioxid mit solchen Sensoren schwierig, da sie meist sehr stark auf Kohlenwasserstoffe oder Ozon reagieren.

Die Reihe der potentiellen Störgase ist dabei umfangreich. Sie umfasst Stickstoffdioxid ($NO_2$), Kohlenmonoxid (CO) und Wasserstoff ($H_2$), Ammoniak ($NH_3$), Ethanol oder Salzsäuren (HCl), Stickstoffmonoxid (NO), Schwefeloxide ($SO_x$), Kohlenoxidsulfid (COS), Lachgas ($N_2O$) und Blausäure (HCN), Wasser ($H_2O$) sowie organische Gase wie Methan, Ethan, Ethen, Acetylen und andere Kohlenwasserstoffe wie Formaldehyd ($CH_2O$). Weitere Störgase sind Amine ($NH_2R_1$, $NH_1R_2$, $N_R3$), Amide ($RC(O)NH_2$, $RC(O)NHR'$, $RC(O)NR'R$), Acrolein ($C_3H_4O$) und Phosgen ($COCl_2$), Aromate wie Benzol ($C_6H_6$), Ethylbenzol, Chlorbenzol, Toluol, Xylol, Styrol und Phenol ($C_6H_6O$). Des Weiteren gibt es Ozon ($O_3$), die große Gruppe der VOCs (volatile organic compounds).

Diese Gase treten teilweise schon in der normalen Umgebungsluft auf, beispielsweise Ozon. Weitere Quellen für Gase sind Brände, Zigarettenrauch, menschliche Aktivität, die Verwendung chemischer Mittel wie Putzmittel, offenstehende Nahrungsmittel oder technische Geräte wie Drucker. Auch Straßenverkehr und sogar die Wetterverhältnisse führen zum Auftreten von Gasen.

Aus der DE 35 26 348 A1 ist ein Kohlendioxidsensor bekannt, der einen auf der Austrittsarbeit beruhenden Gassensor mit primären Aminogruppen zur Ausgabe eines Gasmesswerts und einen Feuchtesensor zur Ausgabe eines Feuchtemesswerts umfasst sowie eine Einrichtung zur Korrektur des Gasmesswerts unter Verwendung des Feuchtemesswerts.

Aus der Schrift von H.-E. Endres et al., "A capacitive CO2 sensor system with suppression of the humidity interference", Sensors and Actuators B 57 (1999), 83-87 ist ein $CO_2$-Sensor bekannt, der auf dem Prinzip einer Kapazitätsmessung beruht. Bei dem offenbarten kapazitiven Sensor wird ein zusätzlicher Feuchtesensor verwendet, um ein Feuchtesignal zu erzeugen.

Ein potentialgesteuerter Feuchtesensor, der hierfür verwendbar ist, ist beispielsweise aus der EP 1 176 418 A2 bekannt. Der potentialgesteuerte Feuchtesensor weist einen gassensitiven Bereich auf, der unabhängig von einer Feuchte polarisierbar ist. Weiterhin weist der gassensitive Bereich eine relative Dielektrizitätskonstante auf, die von der Feuchte abhängig ist.

Nachteilig an den kapazitiven Sensoren aus der Schrift von H.-E. Endres et al. ist, dass eine Beheizung des Sensors nötig ist. Diese Beheizung verbraucht dauerhaft Energie. Weiterhin beeinflusst die erhöhte Temperatur des Sensors gegenüber der Raumtemperatur auch die Dynamik der Interaktionen mit umgebenden Gasen, verändert also mit anderen Worten die Querempfindlichkeiten gegenüber Ziel- und Störgasen gegenüber anderen Sensoren, die weniger stark oder stärker beheizt werden.

Es ist Aufgabe der vorliegenden Erfindung, einen Gassensor anzugeben, der eine Detektion von Kohlendioxid ermöglicht. Eine weitere Aufgabe besteht darin, ein Verfahren zur Erzeugung eines Gasmesswerts, der die Kohlendioxidkonzentration in Luft repräsentiert, anzugeben. Dabei soll jeweils insbesondere der Einfluss von Umgebungsvariablen auf das Messsignal in ausreichender Weise kompensiert werden. Diese Aufgabe wird durch eine Vorrichtung mit den Merkmalen von Anspruch 1 gelöst. Die Aufgabe wird weiterhin durch ein Verfahren mit den Merkmalen von Anspruch 17 gelöst. Die abhängigen Ansprüche betreffen vorteilhafte Ausgestaltungen der Erfindung.

Die erfindungsgemäße Vorrichtung zur Detektion des Kohlendioxidgehalts von Luft weist wenigstens einen Gassensor zur Ausgabe eines Gasmesswerts auf. Der Gassensor weist ein gassensitives Material auf, beispielsweise in Form einer gassensitiven Schicht. Das gassensitive Material ist so gestaltet, dass es auf Kohlendioxid anspricht. Mit anderen Worten ändert sich der Gasmesswert bei einer Änderung der Konzentration von Kohlendioxid in der umgebenden Luft. Dabei ist es zweckmäßig, wenn diese Änderung bei einer Konzentrationsänderung von insbesondere 50 ppm $CO_2$ messbar ist, also die Änderung größer ist als das Signalrauschen. Es ist in einem anderen Beispiel auch ausreichend, wenn die Änderung des Gasmesswerts bei einer Konzentrationsänderung von 500 ppm $CO_2$ messbar ist. Erfindungsgemäß wird bei dem Gassensor der Gasmesswert durch eine Auswertung der Austrittsarbeit des Materials erzeugt.

Es wird hierbei von der Konzentration von Kohlendioxid bzw. Wasser in Luft gesprochen. Darunter wird auch die Messart der meisten gassensitiven Materialien verstanden, bei der nicht ein relativer Anteil eines Gases gemessen wird, sondern das absolute Vorhandensein, d.h. der Partialdruck des Gases. Der Gasmesswert hängt also zweckmäßig vom Partialdruck von Kohlendioxid und Wasser ab.

Erfindungsgemäß ist weiterhin wenigstens ein Feuchtesensor zur Ausgabe eines Feuchtemesswerts vorhanden. Schließlich ist eine Auswerteeinrichtung vorgesehen, die zur Korrektur des Gasmesswerts unter Verwendung des Feuchtemesswerts ausgestaltet ist. Dabei ist die Auswerteeinrichtung zweckmäßig so gestaltet, dass die Korrektur dazu führt, dass der Einfluss einer Änderung der Luftfeuchte auf den Gasmesswert verringert wird.

Erfindungsgemäß weist das gassensitive Material primäre Aminogruppen (R-$NH_2$, R=Rest, z. B. Alkylrest) auf. Diese primären Aminogruppen bilden bei Raumtemperatur bei Vorhandensein von $CO_2$ reversibel geladene Spezies (z. B. Bikarbonat und Carbamat), die zu einer deutlichen Änderung der Austrittsarbeit führen. Materialien mit primären Aminogruppen zeigen eine deutliche reversible Reaktion auf Änderungen des Partialdrucks von Kohlendioxid. Gleichzeitig hat sich gezeigt, dass die Querempfindlichkeiten gegenüber gewissen Störgasen wie beispielsweise $NO_2$, flüchtige Kohlenwasserstoffe oder Lösemittel nicht groß sind.

Es hat sich in Messungen aber vor allem überraschend herausgestellt, dass gerade $CO_2$-Sensoren basierend auf Austrittsarbeitsmessung an dem Material mit primären Aminogruppen unter Umständen auf Luftfeuchte ansprechen, und zwar stärker als auf die vielen sonstigen Störgase. Dies war bereits deswegen unerwartet, weil bei vielen der üblicherweise verwendeten Schichtmaterialien für die Austrittsarbeitsmessung zwar Querempfindlichkeiten auftreten, aber gerade für Wasser kaum. Beispiele sind die Kupferphtalocyanine (CuPC), Bleiphtalocyanine (PbPC) oder sonstige Phtalocyanine, Galliumoxid ($Ga_2O_3$), Platin (Pt) und Titannitrid (TiN).

Für Störgase, die bei primären Aminogruppen eine geänderte Austrittarbeit auslösen, gäbe es wiederum näher liegende Kandidaten. Die Luft enthält nämlich in wechselnder Konzentration Gase, die mit primären Aminen (...-$NH_2$) stark reagieren, wie insbesondere Stickstoffdioxid ($NO_2$), Alkohole (R-OH), Stickstoffmonoxid (NO), Ozon ($O_3$), Wasserstoff ($H_2$), Kohlenmonoxid (CO), Ammoniak ($NH_3$), Salzsäuren (HCl), Blausäure (HCN), Schwefeloxide ($SO_x$), Kohlenoxidsulfid (COS), Lachgas ($N_2O$) und organische Gase.

Dabei zeigt sich der Einfluss von Wasser nicht eindeutig klarer als der Einfluss anderer Quellen auf das Messergebnis. Ähnlich und nicht sofort unterschiedlich ändert sich das Messergebnis infolge von Temperaturänderungen, Verschmutzung, sowie Unvollkommenheiten in der Signalauswertung, Nullpunktsdrift und Hystereseeffekte, d.h. Vorbelastungen aus vorangehenden Messreihen. Daher blieb der überraschend festgestellte Effekt der Luftfeuchte während einer Reihe von Messungen sogar unentdeckt.

Der Feuchtesensor kann ausgestaltet sein, die relative Luftfeuchte zu messen. Er kann auch ausgestaltet sein, die absolute Feuchte zu messen. Dies ist beispielsweise bei der Verwendung optischer Messverfahren der Fall. Es ist vorteilhaft für die Kompaktheit des Aufbaus, beispielsweise polymerbasierte Feuchtesensoren einzusetzen. Diese und andere Feuchtesensoren messen einen Wert, der nicht eindeutig die absolute oder relative Feuchte ist und werden daher mittels eines Temperaturmesswerts korrigiert, um den relativen oder absoluten Wert zu ermitteln. Der Feuchtesensor selbst reagiert zweckmäßig nicht oder nur unwesentlich auf Kohlendioxid.

Bei dem erfindungsgemäßen Verfahren zur Erzeugung eines Gasmesswerts, der die Kohlendioxidkonzentration in Luft repräsentiert, wird ein Gasmesswert durch eine Auswertung der Austrittsarbeit eines Materials durch wenigstens einen Gassensor erzeugt, wobei der Gasmesswert durch die Anwesenheit von Kohlendioxid beeinflusst wird. Weiterhin wird ein Feuchtemesswert von wenigstens einem Feuchtesensor erzeugt. Ferner wird der Gasmesswert unter Verwendung des Feuchtemesswerts so korrigiert, dass der Einfluss der Luftfeuchte auf den Gasmesswert wenigstens verringert ist.

Besonders vorteilhaft ist es, wenn die Reaktion des Gasmesswerts auf eine Änderung der relativen Luftfeuchte um 10 % wenigstens 5 %, insbesondere wenigstens 10 % oder in einem anderen Beispiel wenigstens 20 % der Stärke der Reaktion auf eine Konzentrationsänderung von Kohlendioxid um 1000ppm aufweist.

Als Aufbau für den Gassensor ist ein Feldeffekttransistoraufbau besonders vorteilhaft. Der grundsätzliche Feldeffekttransistoraufbau ist von elektronischen Komponenten her bekannt, d.h. es existieren Drain- und Source-Elektroden und ein leitender Kanal benachbart zu einer Gate-Elektrode. Das besondere am Gassensor mit Feldeffekttransistoraufbau ist, dass das gassensitive Material benachbart zum leitenden Kanal vorgesehen ist. Dadurch beeinflussen elektrische Änderungen im gassensitiven Material die Leitfähigkeit im Kanal.

Die Funktionsweise von Gassensoren auf der Basis von Austrittsarbeitsänderungen bzw. Kontaktpotentialmessungen, wie beispielsweise mittels eines gassensitiven Feldeffekttransistors, beruht auf der physikalischen Tatsache, dass adsorbierte Gasmoleküle an der Materialoberfläche entweder als permanente Dipole vorliegen oder Dipole induzieren. Die Austrittsarbeit des mit Gas bedeckten Materials ändert sich dann um den Potentialsprung an der Dipolschicht auf der Oberfläche. Diese Potentialänderung kann in die Gatespannung eines Feldeffekttransistors eingekoppelt werden, wobei dann als Messgröße die Änderung der Einsatzspannung bei konstantem Strom verwendet werden kann.

Ein solcher Feldeffekttransistoraufbau lässt sich realisieren, indem das gassensitive Material direkt auf die Gate-Elektrode aufgebracht wird. In diesem Fall ist die Herstellung des Sensors in großen Mengen in der mikromechanischen Fertigung möglich. Dabei kann es vorteilhaft sein, wenn das gassensitive Material entweder sehr dünn gemacht wird, oder aber gasdurchlässig gestaltet wird, um eine möglichst große elektrische Wirkung der Gasreaktionen auf den leitenden Kanal zu erzielen.

Besonders vorteilhaft ist der GasFET mit einem Luftspalt zwischen der sensitiven Schicht und dem leitenden Kanal des Feldeffekttransistoraufbaus ausgestattet. Die Realisierung eines Gas-Feldeffekttransistors (GasFET), bei dem zwischen Gate-Elektrode und Kanalbereich des Transistors ein kleiner Luftspalt (0,5 - 5 $\mu$m) vorhanden ist, sieht vor, dass die dem Luftspalt zugewandte Seite der Gate-Elektrode mit dem gassensitiven Material versehen, beispielsweise beschichtet, ist. An der sensitiven Schicht entsteht durch die gasinduzierte Änderung der Elektronenaustrittsarbeit ein zusätzliches Potential in der Größenordnung von typischerweise 10-100 mV, welches als zusätzliche Gatespannung auf den Transistor wirkt.

Ein besonders vorteilhafter Aufbau sieht einen GasFET, insbesondere einen hybriden GasFET mit einer gassensitiven Schicht mit primären Aminogruppen vor.

Die Auslesung der Austrittsarbeit erlaubt hierbei nämlich einen Betrieb bei Raumtemperatur. Dadurch wird nicht nur Energie für eine Beheizung des Sensors gespart, sondern es kann von vornherein der Aufbau vereinfacht werden, da kein Heizer benötigt wird.

Vorteilhaft ist es weiterhin, wenn das gassensitive Material ein Polymer aufweist. Die Stärke der Querempfindlichkeit zur Luftfeuchte von polymerbasierten gassensitiven Schichten hängt von der Art der Auslesung des Signals ab. Gerade beim Übergang von bekannten kapazitiven Sensoren zur Auslesung mittels der Austrittsarbeit, insbesondere mittels eines GasFETs, ergeben sich neben dem überraschenden und erheblichen Vorteil der Messung bei Raumtemperatur auch veränderte Querempfindlichkeiten. So ist die Empfindlichkeit auf Luftfeuchte überraschend stark bei einer Auslesung mittels Austrittsarbeit, wie sie bei GasFETs vorgenommen wird.

Besonders vorteilhaft ist es, wenn die Auswerteeinheit ausgestaltet ist, ein Temperatursignal bei der Korrektur des Gasmesswerts zu berücksichtigen. Zusätzlich kann das Temperatursignal auch bei einer Korrektur des Feuchtesignals berücksichtigt werden. Hierdurch kann ein Einfluss der Umgebungstemperatur auf die Messung verringert werden.

Dies ist besonders vorteilhaft im Zusammenhang mit Sensoren, die bei oder nahe Raumtemperatur betrieben werden, also beispielsweise bei Temperaturen kleiner als 50°C. Dabei ergibt sich generell, aber natürlich am stärksten ausgeprägt bei einem Betrieb bei Raumtemperatur, eine Änderung der Temperatur der Vorrichtung, also der Sensoren, wenn sich die Umgebungstemperatur ändert. Ein ungeheizter Sensor unterliegt mit anderen Worten den Schwankungen der Raumtemperatur, was Änderungen des Adsorptionsverhaltens, chemischen Gleichgewichts u.a. und dadurch bedingt Einfluss auf das Ausgangssignal des Sensors hat.

Sensoren, die bei Raumtemperatur betrieben werden können, sind beispielsweise FET-basierte Gassensoren mit polymerbasierten gassensitiven Schichten mit primären Aminogruppen. Für diese kommt daher eine Temperaturkorrektur besonders zum Tragen.

Gemäß einer vorteilhaften Ausgestaltung der Erfindung weist die Vorrichtung einen Temperatursensor zur Erzeugung des Temperatursignals auf. Temperatursensoren sind einfach und vorgefertigt erhältlich. Ebenso ist es leicht möglich, Temperatursensoren mikromechanisch zu erzeugen, beispielsweise als Widerstandsthermometer mit Dünnschichtbauweise oder als temperaturempfindliche Diode oder sogar als temperaturempfindlichen FET. Damit kann beispielsweise eine monolithische Integration mit dem Gassensor und vorteilhaft auch mit dem Feuchtesensor erreicht werden, was zu einer besonders kleinen und günstigen Bauform führt. Eine ähnliche, vorteilhafte Möglichkeit ist es, einen Feuchtesensor vorzusehen, der selbst bereits ein Temperatursignal erzeugt und dieses Temperatursignal mitzuverwenden, also beispielsweise der Auswerteeinheit zur Verfügung zu stellen.

Es ist aber in einer vorteilhaften Alternative auch möglich, anstelle oder ggf. zusätzlich zum Temperatursensor eine Einrichtung zur Beheizung des Gassensors und/oder des Feuchtesensors vorzusehen. Damit kann beispielsweise eine Beheizung vorgenommen werden. Wird diese beispielsweise auf 50°C oder 40°C begrenzt und geregelt, ist der Sensor automatisch im Wesentlichen unabhängig von der Umgebungstemperatur. Gleichzeitig kann die Beheizungseinrichtung auch ausgestaltet sein, die Funktion der Bestimmung des Temperatursignals mitzuerfüllen. Dazu kann beispielsweise ein Dünn- oder Dickschichtheizer in Form eines Heizmäanders zum Einsatz kommen. Eine Möglichkeit hierzu besteht darin, die zum Halten der vorgegebenen Temperatur nötige Heizleistung als Maß für die Umgebungstemperatur zu verwenden. Wird mehr Heizleistung benötigt, so ist der Wärmeabtransport größer, d.h. entweder der Sensor ist einer Luftströmung ausgesetzt oder es ist kälter. In einem Gehäuse ist eine Luftströmung unterbunden, so dass dieser Einfluss ausgeschlossen werden kann.

Schließlich besteht eine weitere Alternative zur Erzeugung des Temperatursignals darin, den Gassensor und/oder den Feuchtesensor mittels der Beheizungseinrichtung unterschiedlichen Temperaturen auszusetzen. Die Auswerteeinrichtung ist dann zweckmäßig so ausgestaltet, dass sie aus den Reaktionen von Gassensor und/oder Feuchtesensor auf die Umgebungstemperatur schließt.

Um Einflüsse des Drucks ausgleichen zu können, ist in einer weiteren Ausgestaltung der Erfindung ein Drucksensor vorgesehen. Der Luftdruck kann beispielsweise durch die Höhe über dem Meeresspiegel und durch Wettereinflüsse variieren. Besonders vorteilhaft ist es, wenn der Drucksensor monolithisch mit dem Gassensor und/oder den weiteren vorhandenen Sensoren realisiert ist, d.h. auf einem gemeinsamen Substrat erzeugt.

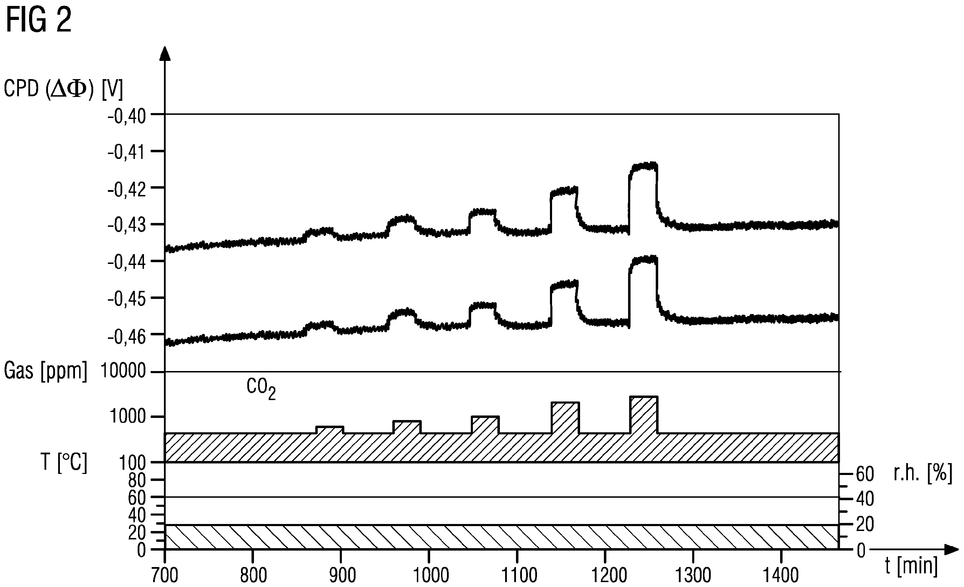

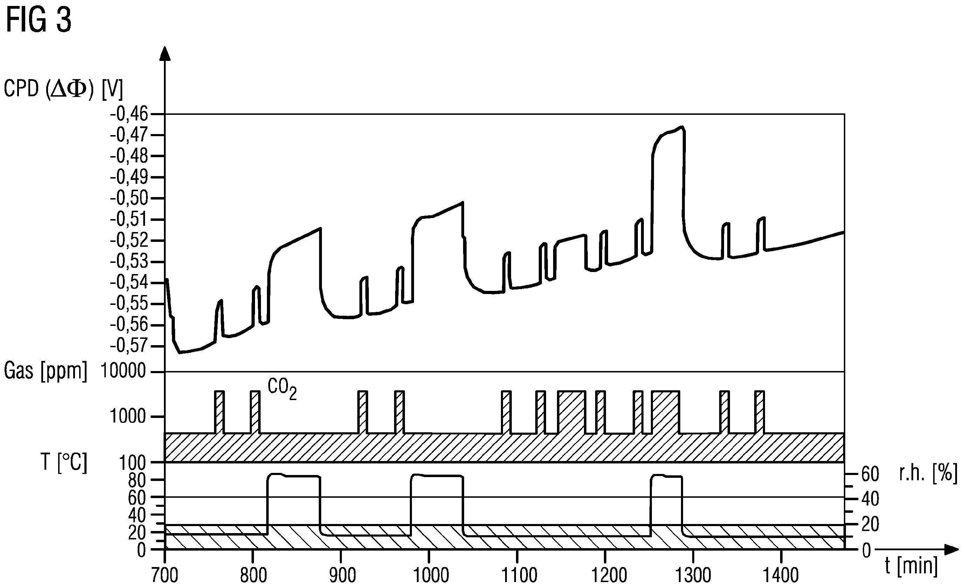

Der Einfluss der Luftfeuchte auf den Gasmesswert lässt sich in 2 Wirkungen aufteilen. Die erste Wirkung entspricht der Funktion, die das gassensitive Material als Feuchtesensor haben könnte. Sie ist völlig unabhängig vom Vorhandensein von Kohlendioxid. Das ist in Fig. 2 dargestellt. Der Wechsel von 30 % r.h. (relative Luftfeuchte) auf 0 % r.h. stellt einen Extremfall dar, die ebenfalls in Fig. 2 dargestellten Wechsel zwischen 10 % r.h. und 60 % r.h. können jedoch unter Umständen auftreten und ergeben bei dieser Sensorschicht ein Sensorsignal, das größer ist als das Nutzsignal bei Wechsel von 400 auf 4000 ppm $CO_2$. Beide Signale überlagern sich in nahezu linearer Form. Dabei ist zu beachten, dass die Sensorantworten der drei Messungen (Abbildung 3 a,b,c) nicht direkt miteinander vergleichbar sind, da für die Messungen unterschiedliche Sensorschichtdicken verwendet worden sind.

Die zweite Wirkung besteht darin, dass die Größe des Messeffekts auf $CO_2$ etwas von der jeweils herrschenden Luftfeuchtigkeit abhängt, d.h. das durch beispielsweise eine Konzentrationsänderung von 100 ppm Kohlendioxid hervorgerufene Signal ist abhängig vom Wasserpartialdruck. So kann eine Konzentrationsänderung von 100 ppm Kohlendioxid bei einer Luftfeuchte von 30 % beispielsweise eine Änderung des elektrischen Signals von 10 mV erzeugen, während die gleiche Konzentrationsänderung bei 70 % Luftfeuchte eine Änderung des elektrischen Signals von 13 mV erzeugt. Dabei ist der Unterschied von 40 % Luftfeuchte, der selbst beispielsweise eine Signaländerung von 40 mV erzeugt, bereits berücksichtigt und korrigiert. D.h., selbst, wenn die erste Wirkung, die weiter oben erläutert ist, vollständig korrigiert wird, verbleibt die zweite Wirkung und kann ebenfalls korrigiert werden, um die Genauigkeit der Kohlendioxid-Messung zu erhöhen.

In einer vorteilhaften Ausgestaltung der Erfindung wird die erste Wirkung korrigiert. Dies kann beispielsweise anhand von tabellarisch vorhandenen Korrekturwerten geschehen. Es ist auch möglich, die Korrektur analytisch mit hinterlegten Koeffizienten durchzuführen.

Erfindungsgemäß wird die zweite Wirkung korrigiert. Dabei wird also nicht nur der durch Feuchte bewirkte Ausschlag des Gasmesswerts herausgerechnet, sondern zusätzlich der übrigbleibende Gasmesswert, der dann vom Kohlendioxid stammt, anhand der Feuchte korrigiert. Dies kann ebenfalls beispielsweise anhand von tabellarisch vorhandenen Korrekturwerten geschehen. Es ist auch möglich, die Korrektur analytisch mit hinterlegten Koeffizienten durchzuführen.

Ist bei einer beispielhaften Ausführung in einem Mikroprozessor genügend Speicherplatz vorhanden, können beide Wirkungen auch zusammen korrigiert werden, indem beispielsweise eine zweidimensionale Tabelle von Werten hinterlegt wird. Die Tabelle liefert dann anhand des Gasmesswerts und des Feuchtemesswerts einen für beide Wirkungen korrigierten Wert für die Konzentration von Kohlendioxid.

Vorteilhaft ist es, wenn der Einfluss einer unterschiedlichen Ansprechzeit des Feuchtesensors und Gassensors berücksichtigt wird. Dies kann beispielsweise dadurch geschehen, dass geprüft wird, ob die zeitliche Änderung des Gasmesswerts und/oder des Feuchtemesswerts einen festlegbaren Schwellwert überschreitet, und in diesem Fall der Gasmesswert erst nach Ablauf einer festlegbaren Wartezeit berücksichtigt wird. So kann beispielsweise bei starken Signaländerungen, bewirkt durch das Öffnen eines Fensters o.ä., eine Wartezeit von 2 Minuten eingehalten werden, und die weiteren Messwerte erst danach wieder als verlässlich eingeschätzt werden. Die Messwerte innerhalb der Wartezeit werden dann zweckmäßig verworfen.

Dabei ist es vorteilhaft, wenn der Gasmesswert und/oder der Feuchtemesswert so bearbeitet wird, dass Signalschwankungen, die nicht von Schwankungen der Gaskonzentrationen bewirkt werden, nicht berücksichtigt werden bei der Ermittlung der zeitlichen Änderung.

Hierzu kann beispielsweise die Bildung eines laufenden Mittelwerts des letzten drei, fünf, oder einer anderen Anzahl an Messwerten dienen. Erst dieser gleitende Mittelwert wird auf seine zeitliche Änderung geprüft. Weitere Methoden, um verbesserte Werte für die zeitliche Änderung zu bekommen, bestehen in einer Filterung der Messwerte. Die Filterung kann analog geschehen, beispielsweise sensornah durch analoge elektronische Komponenten. Dabei können Filter beispielsweise erster oder zweiter oder einer anderen Ordnung verwendet werden. Vorteilhafterweise funktioniert die analoge Filterung aus Sicht eines Mikroprozessors zur Verarbeitung der Messwerte passiv, d.h. die später digitalisierten Messwerte sind bereits verbessert ohne weiteren Aufwand im Mikroprozessor.

Die Filterung kann aber auch digital erfolgen. Dazu werden die digitalisierten Messwerte beispielsweise im Mikroprozessor bearbeitet, um die Filterung zu erreichen. Hierbei ist eine größere Flexibilität bei der Verarbeitung möglich, da die Messwerte zunächst unverändert zur Verfügung stehen.

Es ist auch möglich, das Ansprechverhalten des gassensitiven Materials in Abhängigkeit des jeweils vorliegenden Feuchteniveaus in der Auswerteeinrichtung zu speichern. Dann wird eine auftretende Änderung des Gasmesswertes in Abhängigkeit des Feuchtemesswerts bewertet. So wird beispielsweise bei kleinen Feuchten die Änderung des Gasmesswertes zuerst stärker bewertet und dann gemäß den abgelegten Zeitkonstanten für das Ansprechen des Gasmesswertes dann die verstärkte Bewertung entsprechend zurückgenommen. Das Ansprechverhalten kann z. B. gut gemäß einer Exponentialfunktion mit feuchteabhängiger Zeitkonstante beschrieben werden. Dann wird der Gasmesswert bei einer Änderung entsprechend der inversen Exponentialfunktion zeitabhängig verstärkt bewertet, um dann nach dem einfachen oder vielfachen der Zeitkonstanten dann diese verstärkte Bewertung wieder aufzuheben. Vorteilhaft wird dabei eine Exponentialfunktion verwendet, deren Betrag gegen Null abfällt, also eine Funktion der Form

$$K = K_F \cdot e^{-(t-t_0)/T_F}$$

Dabei sind

K       ein Korrekturwert zur Korrektur des Gasmesswerts

$K_F$      ein Feuchtabhängiger Vorfaktor

$T_F$      eine Feuchteabhängige Zeitkonstante

$t-t_0$    die seit Beginn der Korrektur des Ansprechverhaltens abgelaufene Zeit

In diesem Fall strebt K von selbst mit der Zeit gegen Null. Das bedeutet, dass die Korrektur mit ablaufender Zeit von selbst schwächer wird. Das ist sinnvoll, da ein Fehler im Gasmesswert durch das unterschiedlich schnelle Ansprechen mit der Zeit ebenfalls kleiner wird und bei genügend großer verstrichener Zeit ganz verschwindet.

Es ist auch in Ausgestaltung der Erfindung möglich, dass die Auswerteeinrichtung, beispielsweise der Mikroprozessor bei der Korrektur des Ansprechverhaltens die Temperatur mitberücksichtigt.

Figur 1     einen Aufbau für einen Kohlendioxidsensor als SGFET,

Figur 2     ein Messergebnis einer AMO/PTMS-Schicht für Kohlendioxid,

Figur 3     ein Messergebnis einer AMO/PTMS-Schicht für $CO_2$ und Wasser,

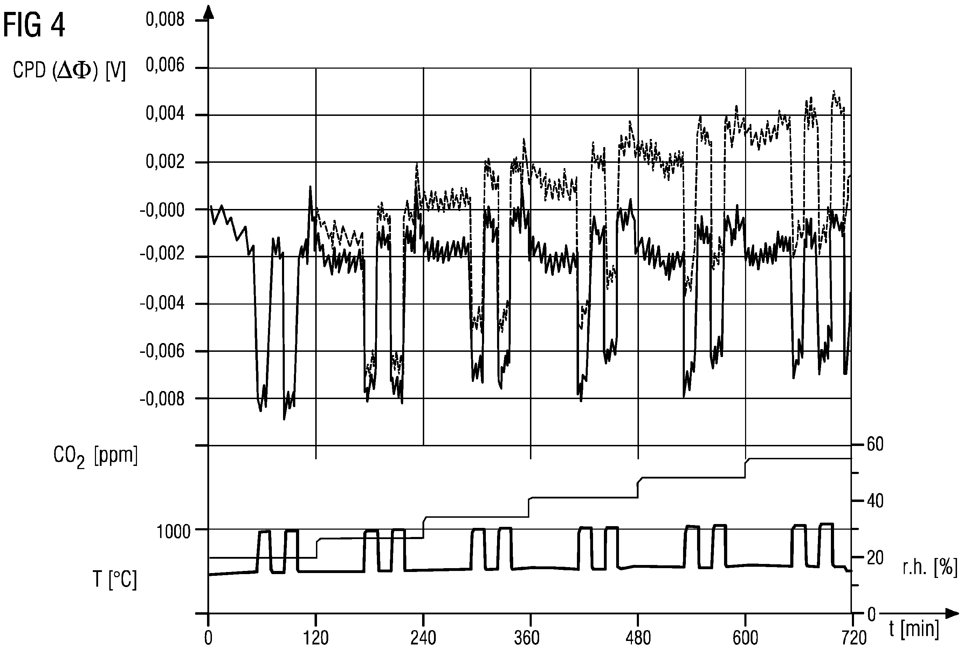

Figur 4     ein Messergebnis einer AMO/PTMS-Schicht mit Feuchtekompensation der Grundlinie und

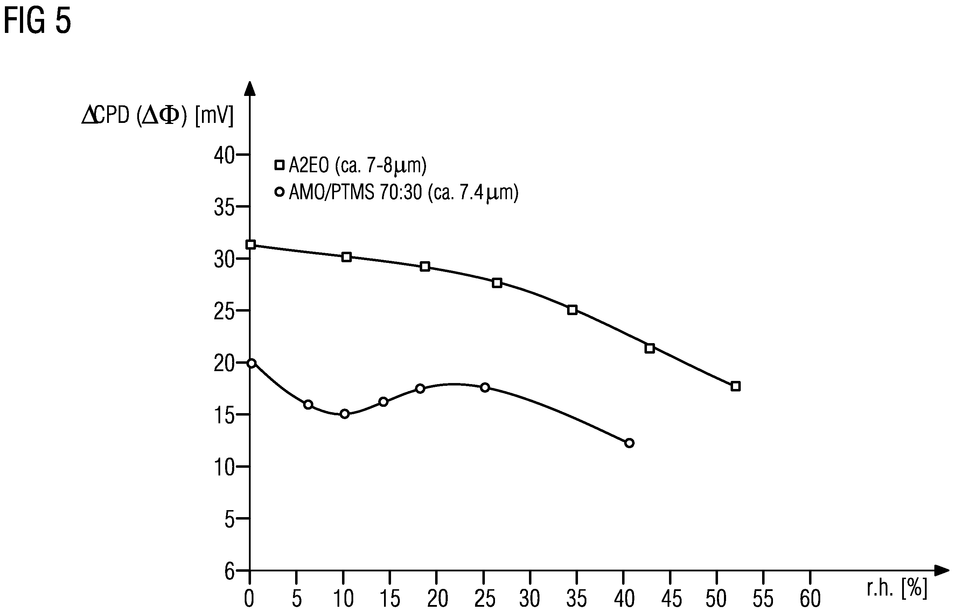

Figur 5     ein Messergebnis von Polymerschichten für die Feuchteabhängigkeit des Kohlendioxidmesssignals.

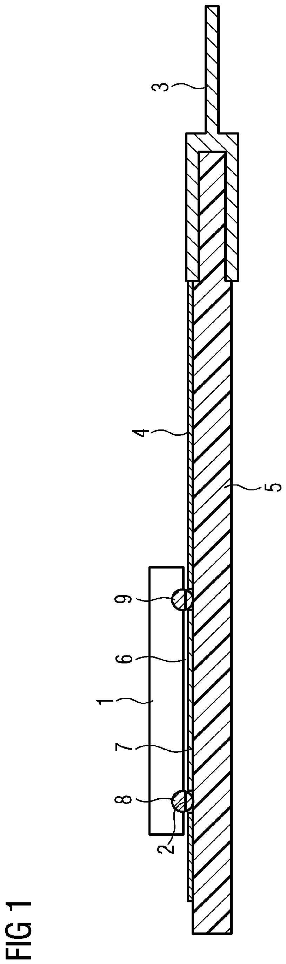

Die Fig. 1 zeigt den grundsätzlichen Aufbau eines gassensitiven FETs gemäß einem Beispiel für einen erfindungsgemäßen Aufbau. Diese umfasst einen CMOS-Transistor 1 mit einer Source-Elektrode 8 und einer Drain-Elektrode 9. Dabei wird eine FET-Struktur in Form des CMOS-Transistors 1 in Flip-Chip-Technik auf ein mit Leiterbahnen 4 versehenes Keramiksubstrat 5 montiert. Dies kann beispielsweise mittels eines Leitklebstoffes 2 geschehen. Die gassensitive Schicht 7 ist partiell auf dem Keramiksubstrat 5 aufgebracht und mit den Leiterbahnen 4 entsprechend kontaktiert. Der Gaskanal ist der Luftspalt 6 zwischen Gate und CMOS-Transistor. Das Keramiksubstrat 5 dient als Träger der gassensitiven Schicht und gleichzeitig als Träger des gesamten Sensoraufbaus, sodass in diesem Beispiel kein Einbau in einen Sensorsockel notwendig ist. Auf dieses Keramiksubstrat 5 können Steckstifte 3 angebracht werden, sodass das elektronische Bauelement direkt beispielsweise in eine Singleinline-Steckverbindung eingebracht werden kann. Alternativ sind auch andere Ausführungen möglich, beispielsweise die Ausführung als SMD-Bauelement (surface mounted device).

Ein erster Sensor, dessen Messergebnis in Figur 2 dargestellt ist, weist eine sog. AMO/PTMS-Schicht als Sensorschicht auf. Dieses Materialsystem wird auch als Heteropolysiloxan bezeichnet, da hier das Material aus zwei verschiedenen Ausgangssilanen gebildet wird. Zur Herstellung dieser Schicht wird Aminopropyltrimethoxysilan (AMO) und Propyltrimethoxysilan (PTMS) in Methanol gelöst. Die Lösung wird in einem Glaskolben unter Zusatz einer geringen Menge Wasser 3 Stunden unter Rückfluss gekocht. Die entstehende Lösung wird nach dem Abkühlen mittels eines Spin-Coating-Prozesses auf ein Substrat (z. B. mit Gold beschichtetes $Al_2O_3$-Keramik) aufgebracht und im Ofen in Stickstoffatmosphäre bei 120°C sechzehn Stunden lang ausgehärtet. Die so erzeugte Schicht weist in diesem Beispiel eine Dicke von 12,8 $\mu$m auf.

Figur 2 zeigt zwei Messergebnisse an der so erhaltenen Sensorschicht mittels einer Kelvinsonde. Während der Messdauer wurde der erste Sensor auf Raumtemperatur betrieben, also ohne Beheizung. Der erste Sensor weist keine Einrichtung zur Beheizung auf. Die künstlich erzeugte Gasumgebung der Sensorschicht wies eine relative Feuchte von 40 % auf. Während der mehrstündigen Messung wurde die Konzentration von Kohlendioxid von einem Grundniveau von ca. 400 ppm in Intervallen stufenweise angehoben und wieder auf das Grundniveau zurückgesetzt. Die kleinste erzeugte erhöhte Konzentration lag bei ca. 600 ppm, also ca. 200 ppm über dem Grundniveau. Die höchste erzeugte Konzentration lag dabei bei ca. 4000 ppm.

Das Messsignal CPD (contact potential difference) zeigt einen deutlichen Ausschlag bei einer Konzentration von 4000 ppm $CO_2$. Bei geringeren Konzentrationserhöhungen ist das Signal entsprechend schwächer. Auch bei der geringsten Konzentrationserhöhung von ca. 200 ppm ist das Signal deutlich erkennbar.

Eine zweite Ausführungsmöglichkeit für die gassensitive Schicht besteht in einer Cysteamin-Schicht. Zur Herstellung dieser Schicht wird eine Cysteamin-Lösung auf eine Goldoberfläche eines Kelvinsubstrats aufgetropft. Zur Ausbildung von Thiol-Gold-Bindungen wird die Probe zwei Stunden bei Raumtemperatur stehen gelassen. Anschließend wird die Cysteamin-Lösung mit Wasser abgespült und das Substrat im Stickstoffstrom getrocknet.

Eine dritte Ausführungsmöglichkeit für die gassensitive Schicht besteht in einer sog. AMO-Schicht als Sensorschicht. Dieses Materialsystem wird auch als Polysiloxan bezeichnet, da hier das Material durch Polymerisation eines Siloxans gebildet wird. Zur Herstellung dieser Schicht wird Aminopropyltrimethoxysilan (AMO) in Methanol gelöst. Die Lösung wird in einem Glaskolben unter Zusatz einer geringen Menge Wasser drei Stunden unter Rückfluss gekocht. Die entstehende Lösung wird nach dem Abkühlen mittels eines Spin-Coating-Prozesses auf ein Substrat aufgebracht und im Ofen in Stickstoffatmosphäre bei 120°C sechzehn Stunden lang ausgehärtet. Die so erzeugte Schicht weist in diesem Beispiel eine Dicke von 3,9 $\mu$m auf.

Figur 3 zeigt ein Messergebnis an einer AMO-Sensorschicht. Der Aufbau des Sensors ist in Figur 1 gezeigt. Während der Messdauer wurde der Sensor auf Raumtemperatur betrieben, also ohne Beheizung. Die künstlich erzeugte Gasumgebung der Sensorschicht wurde so gesteuert, dass sowohl die Feuchte als auch die Kohlendioxidkonzentration variiert wurde. Es ist erkennbar, dass die Sensorschicht deutliche Reaktionen auf beiderlei Änderungen zeigt. Der Einfluss der Luftfeuchte ist nicht vernachlässigbar.

In einem Ausführungsbeispiel für einen konkreten Sensoraufbau weist dieser neben einem FET-basierten AMO-Gassensor einen Feuchtesensor und einen als Dünnschichtmetallisierung ausgebildeten Temperatursensor auf. Eine Auswerteelektronik, die sowohl mit dem Gasmesschip integriert sein kann also auch außerhalb realisiert sein kann, nimmt die Signale des Feuchtesensors, des Temperatursensors und des Gassensors auf. Der vom Gassensor gelieferte Gasmesswert wird dann mittels des Feuchtesignals korrigiert.

Dies geschieht aufgrund von in der Elektronik hinterlegten Kennlinien für die jeweiligen Abhängigkeiten. In einem ersten Schritt wird ein entsprechender Wert vom Sensorsignal addiert oder subtrahiert. Dieser Schritt korrigiert im Allgemeinen schon den größten Teil der Querempfindlichkeit. Hierdurch wird der Einfluss der Feuchte korrigiert, bei dem der gerade vorliegende Kohlendioxidgehalt der Luft unerheblich ist, insoweit also Kohlendioxid- und Feuchtesignal unabhängig voneinander sind.

In einem zweiten Schritt wird dann die Änderung des Sensorsignals zur $CO_2$-Bestimmung mit einem Faktor entsprechend der hinterlegten Kennlinie korrigiert. In diesem zweiten Schritt wird also der Fehler im Messsignal korrigiert, der sich aus der Interaktion zwischen Wasser und $CO_2$ ergibt.

In einer weiteren Ausführungsform wird zusätzlich zur Korrektur der Feuchteabhängigkeit die Temperaturabhängigkeit des Sensorsignals mit Hilfe des Temperatursensors kompensiert. Dazu wird im ersten Schritt zuerst der Feuchte- und der Temperatureinfluss auf das Sensorgrundsignal kompensiert. Dann wird wieder gemäß dem zweiten Schritt der Sensorausschlag ermittelt und gemäß einem abgelegten Kennfeld wieder auf der Basis des herrschenden Feuchtigkeits- und Temperaturniveaus bewertet.

Dieses Vorgehen kann kontinuierlich angewendet werden, wenn gleiche Ansprechkinetik der beiden Sensoren auf Temperatur und Feuchtewechsel vorliegt. Wenn diese nicht ideal gegeben ist, wird beispielsweise die folgende Vorgehensweise angewendet. Anhand des Signals des Temperatur- bzw. Feuchtesensors wird festgestellt, dass eine der Größen sich signifikant (über einen gewissen Schwellwert, von dem bekannt ist, dass er einen Signalhub hervorruft, der einen Einfluss auf die $CO_2$ Messung hat) verändert hat. Als Reaktion wird dann zur Signalausgabe solange gewartet, bis die bekannten Ansprechzeiten des $CO_2$ und/der des Feuchtesensors vorbei sind und erst dann wieder ein $CO_2$-Signal ausgegeben. Dadurch ist sichergestellt, dass nach z. B. einem schnellen Feuchtewechsel dem $CO_2$-Wert wieder vertraut werden darf.

**Patentansprüche**

1. Vorrichtung zur Detektion des Kohlendioxidgehalts von Luft mit:

  - wenigstens einem Gassensor zur Ausgabe eines Gasmesswerts,
  - wenigstens einem Feuchtesensor zur Ausgabe eines Feuchtemesswerts, und

- einer Einrichtung zur Korrektur des Gasmesswerts unter Verwendung des Feuchtemesswerts, wobei der Gassensor ein gassensitives Material (7) aufweist, das auf Kohlendioxid anspricht und primäre Aminogruppen aufweist, und wobei der Gassensor ausgestaltet ist, den Gasmesswert durch eine Auswertung der Austrittsarbeit des Materials zu erzeugen, und mit Mitteln zur Korrektur des Gasmesswerts des Gassensors, derart ausgestaltet, den Einfluss der Feuchte auf den Gasmesswert unabhängig vom Kohlendioxidgehalt zu korrigieren, **gekennzeichnet dadurch, dass** ein Wert aus hinterlegten Kennlinien zum Gasmesswert addiert oder subtrahiert wird, und zusätzlichen Mitteln zur Korrektur des Gasmesswerts des Gassensors, derart ausgestaltet, den Einfluss der Luftfeuchte auf den Einfluss von Kohlendioxid auf den Gasmesswert zu korrigieren, indem der Gasmesswert mit einem Faktor anhand der hinterlegten Kennlinien korrigiert wird.

2. Vorrichtung gemäß Anspruch 1, bei der das gassensitive Material (7) ein Polymer aufweist.

3. Vorrichtung gemäß Anspruch 1 oder 2 mit einem Temperatursensor, wobei weiterhin die Einrichtung zur Korrektur des Gasmesswerts ausgestaltet ist, eine Temperaturabhängigkeit des Gasmesswerts mit Hilfe des Temperatursensors zu kompensieren.

4. Vorrichtung gemäß einem der vorangehenden Ansprüche, bei der der Gassensor einen Feldeffekttransistoraufbau (1, 8, 9) mit einem Luftspalt (6) zwischen dem gassensitiven Material (7) und dem leitenden Kanal des Feldeffekttransistoraufbaus besitzt.

5. Vorrichtung gemäß einem der vorangehenden Ansprüche mit einem Drucksensor.

6. Vorrichtung gemäß einem der vorangehenden Ansprüche mit einer Einrichtung zur Beheizung des Gassensors und/oder des Feuchtesensors.

7. Vorrichtung gemäß Anspruch 6, ausgestaltet zur Ermittlung eines Temperatursignals mit der Beheizungsvorrichtung.

8. Vorrichtung gemäß Anspruch 7, ausgestaltet, das Temperatursignal anhand der Reaktion des Gasmesswerts und/oder Feuchtemesswerts auf verschiedene Beheizungsstufen zu ermitteln.

9. Vorrichtung gemäß Anspruch 7, ausgestaltet, das Temperatursignal anhand der Leistungsaufnahme der Beheizungsvorrichtung zum Halten einer konstanten Temperatur zu ermitteln.

10. Vorrichtung gemäß einem der vorangehenden Ansprüche mit Mitteln zur Korrektur des Gasmesswerts des Gassensors, derart ausgestaltet, dass der Einfluss einer unterschiedlichen Ansprechzeit des Feuchtesensors und Gassensors korrigiert wird.

11. Vorrichtung gemäß Anspruch 10, bei der die Mittel zur Korrektur des Gasmesswerts des Gassensors ausgestaltet sind, eine Korrektur des Einflusses der unterschiedlichen Ansprechzeit vorzunehmen, indem dem Gasmesswert eine positive oder negative Korrekturgröße hinzugerechnet wird, wobei der Betrag der Korrekturgröße einen exponentiell abfallenden Verlauf aufweist.

12. Vorrichtung gemäß einem der vorangehenden Ansprüche, derart ausgestaltet, dass geprüft wird, ob die zeitliche Änderung des Gasmesswerts des Gassensors und/oder des Feuchtemesswerts einen festlegbaren Schwellwert überschreitet, und in diesem Fall der Gasmesswert erst nach Ablauf einer festlegbaren Wartezeit berücksichtigt wird.

13. Vorrichtung gemäß einem der vorangehenden Ansprüche, bei der das gassensitive Material ausgestaltet ist, sowohl auf Kohlendioxid als auch auf Luftfeuchte anzusprechen.

14. Verfahren zur Erzeugung eines Gasmesswerts, der die Kohlendioxidkonzentration in Luft repräsentiert, bei dem

- ein Gasmesswert durch eine Auswertung der Austrittsarbeit eines Materials (7) mit primären Aminogruppen durch wenigstens einen Gassensor erzeugt wird, wobei der Gasmesswert durch die Anwesenheit von Kohlendioxid beeinflusst wird,
- ein Feuchtemesswert von wenigstens einem Feuchtesensor erzeugt wird,
- der Gasmesswert unter Verwendung des Feuchtemesswerts so korrigiert wird, dass der Einfluss der Luftfeuchte auf den Gasmesswert wenigstens verringert ist,

wobei der Gasmesswert des Gassensors anhand von hinterlegten Kennlinien derart korrigiert wird, dass der Einfluss der Feuchte auf den Gasmesswert unabhängig vom Kohlendioxidgehalt korrigiert wird, **dadurch gekennzeichnet, dass** ein Wert aus den hinterlegten Kennlinien zum Gasmesswert addiert oder subtrahiert wird, und der Einfluss der Luftfeuchte auf den Einfluss von Kohlendioxid auf den Gasmesswert korrigiert wird, indem der Gasmesswert mit einem Faktor anhand der hinterlegten Kennlinien korrigiert wird.

**Claims**

1. Apparatus for detecting the carbon dioxide content of air, comprising:

   - at least one gas sensor for outputting a gas measurement value,
   - at least one humidity sensor for outputting a humidity measurement value, and
   - a device for correcting the gas measurement using the humidity measurement value, wherein the gas sensor comprises a gas-sensitive material (7) which responds to carbon dioxide and comprises primary amino groups, and wherein the gas sensor is configured for generating the gas measurement value by means of an evaluation of the work function of the material,

   and comprising means for correcting the gas measurement value of the gas sensor, configured in such a way as to correct the influence of the humidity on the gas measurement value independently of the carbon dioxide content, **characterized in that** a value from stored characteristic curves is added to or subtracted from the gas measurement value, and additional means for correcting the gas measurement value of the gas sensor, configured in such a way as to correct the influence of the air humidity on the influence of carbon dioxide on the gas measurement value by virtue of the gas measurement value being corrected with a factor on the basis of the stored characteristic curves.

2. Apparatus according to Claim 1, wherein the gas-sensitive material (7) comprises a polymer.

3. Apparatus according to Claim 1 or 2 comprising a temperature sensor, wherein the device for correcting the gas measurement value is furthermore configured for compensating for a temperature dependence of the gas measurement value with the aid of the temperature sensor.

4. Apparatus according to any of the preceding claims, wherein the gas sensor has a field effect transistor construction (1, 8, 9) with an air gap (6) between the gas-sensitive material (7) and the conducting channel of the field effect transistor construction.

5. Apparatus according to any of the preceding claims comprising a pressure sensor.

6. Apparatus according to any of the preceding claims comprising a device for heating the gas sensor and/or the humidity sensor.

7. Apparatus according to Claim 6, configured for determining a temperature signal with the heating apparatus.

8. Apparatus according to Claim 7, configured for determining the temperature signal on the basis of the reaction of the gas measurement signal and/or humidity signal to different heating levels.

9. Apparatus according to Claim 7, configured for determining the temperature signal on the basis of the power consumption of the heating apparatus for maintaining a constant temperature.

10. Apparatus according to any of the preceding claims comprising means for correcting the gas measurement value of the gas sensor, configured in such a way that the influence of a different response time of the humidity sensor and gas sensor is corrected.

11. Apparatus according to Claim 10, wherein the means for correcting the gas measurement value of the gas sensor are configured for performing a correction of the influence of the different response time by adding a positive or negative correction variable to the gas measurement value, wherein the absolute value of the correction variable has an exponentially falling profile.

12. Apparatus according to any of the preceding claims, configured in such a way that a check is made to determine whether the temporal change in the gas measurement value of the gas sensor and/or in the humidity measurement value exceeds a definable threshold value, and in this case the gas measurement value is taken into account only after a definable waiting time has elapsed.

13. Apparatus according to any of the preceding claims, wherein the gas-sensitive material is configured for responding both to carbon dioxide and to air humidity.

14. Method for generating a gas measurement value representing the carbon dioxide concentration in air, wherein

    - a gas measurement value is generated by an evaluation of the work function of a material (7) comprising primary amino groups by means of at least one gas sensor, wherein the gas measurement value is influenced by the presence of carbon dioxide,
    - a humidity measurement value is generated by at least one humidity sensor,
    - the gas measurement value is corrected using the humidity measurement value in such a way that the influence of the air humidity on the gas measurement value is at least reduced,

wherein the gas measurement value of the gas sensor is corrected on the basis of stored characteristic curves in such a way that the influence of the humidity on the gas measurement value is corrected independently of the carbon dioxide content, **characterized in that** a value from the stored characteristic curves is added to or subtracted from the gas measurement value, and the influence of the air humidity on the influence of carbon dioxide on the gas measurement value is corrected by virtue of the gas measurement value being corrected with a factor on the basis of the stored characteristic curves.

## Revendications

1. Dispositif pour la détection de la teneur en dioxyde de carbone de l'air avec :

    - au moins un détecteur de gaz pour la production d'une valeur de mesure de gaz,
    - au moins un détecteur d'humidité pour la production d'une valeur de mesure d'humidité, et
    - un moyen pour la correction de la valeur de mesure de gaz par utilisation de la valeur de mesure d'humidité, dans lequel le détecteur de gaz comprend un matériau sensible au gaz (7) qui concerne le dioxyde de carbone et qui comprend des groupes amine primaire, et dans lequel le détecteur de gaz est conçu pour élaborer la valeur de mesure de gaz par une évaluation du travail d'extraction du matériau,

    et avec des moyens pour la correction de la valeur de mesure de gaz du détecteur de gaz qui sont conçus pour corriger l'influence de l'humidité sur la valeur de mesure de gaz indépendamment de la teneur en dioxyde de carbone, **caractérisé en ce qu'**une valeur prise sur les courbes caractéristiques enregistrées est ajoutée à la valeur de mesure de gaz ou en est soustraite, et des moyens supplémentaires pour la correction de la valeur de mesure de gaz du détecteur de gaz conçus pour corriger l'influence de l'humidité de l'air sur l'influence du dioxyde de carbone sur la valeur de mesure de gaz, du fait que la valeur de mesure de gaz est corrigée par un facteur à l'aide des courbes caractéristiques enregistrées.

2. Dispositif selon la revendication 1, dans lequel le matériau sensible au gaz (7) comprend un polymère.

3. Dispositif selon la revendication 1 ou la revendication 2 avec un capteur de température, dans lequel le moyen pour la correction de la valeur de mesure de gaz est en outre conçu pour compenser une dépendance en température de la valeur de mesure de gaz à l'aide du capteur de température.

4. Dispositif selon l'une des revendications précédentes, dans lequel le détecteur de gaz possède une structure de transistor à effet de champ (1, 8, 9) avec un espace d'air (6) entre le matériau sensible au gaz (7) et le canal de conduction de la structure de transistor à effet de champ.

5. Dispositif selon l'une des revendications précédentes avec un capteur de pression.

6. Dispositif selon l'une des revendications précédentes avec un moyen pour le chauffage du détecteur de gaz et/ou du détecteur d'humidité.

7. Dispositif selon la revendication 6, conçu pour la détermination d'un signal de température avec le dispositif de chauffage.

8. Dispositif selon la revendication 7, conçu pour déterminer le signal de température à l'aide de la réaction de la valeur de mesure de gaz et/ou de la valeur de mesure d'humidité en différents stades de chauffage.

9. Dispositif selon la revendication 7, conçu pour déterminer le signal de température à l'aide de la consommation en énergie du dispositif de chauffage pour le maintien d'une température constante.

10. Dispositif selon l'une des revendications précédentes avec des moyens pour la correction de la valeur de mesure de gaz du détecteur de gaz, conçus de telle sorte que l'influence d'un temps de réponse différent du détecteur d'humidité et du détecteur de gaz soit corrigée.

11. Dispositif selon la revendication 10, dans lequel les moyens pour la correction de la valeur de mesure de gaz du détecteur de gaz sont conçus pour effectuer une correction de l'influence des différents temps de réponse, du fait qu'une grandeur de correction positive ou négative est ajoutée à la valeur de mesure de gaz, dans lequel la valeur de la grandeur de correction comprend une décroissance exponentielle.

12. Dispositif selon l'une des revendications précédentes, conçu de telle sorte qu'il soit vérifié si la variation temporelle de la valeur de mesure de gaz du détecteur de gaz et/ou de la valeur de mesure d'humidité dépasse une valeur de seuil pouvant être définie, et que dans ce cas la valeur de mesure de gaz ne soit prise en considération qu'après l'expiration d'un temps d'attente pouvant être défini.

13. Dispositif selon l'une des revendications précédentes, dans lequel le matériau sensible au gaz est conçu aussi bien pour concerner le dioxyde de carbone que l'humidité de l'air.

14. Procédé pour l'obtention d'une valeur de mesure de gaz qui représente la concentration du dioxyde de carbone dans l'air, dans lequel

- une valeur de mesure de gaz est obtenue par une évaluation du travail d'extraction d'un matériau (7) avec des groupes amine primaire à l'aide d'au moins un détecteur de gaz, dans lequel la valeur de mesure de gaz est influencée par la présence de dioxyde de carbone,
- une valeur de mesure d'humidité est obtenue par au moins un détecteur d'humidité,
- la valeur de mesure de gaz est corrigée par utilisation de la valeur de mesure d'humidité de sorte que l'influence de l'humidité de l'air sur la valeur de mesure de gaz soit au moins diminuée,

dans lequel la valeur de mesure de gaz du détecteur de gaz est corrigée à l'aide de courbes caractéristiques enregistrées de telle sorte que l'influence de l'humidité sur la valeur de mesure de gaz soit corrigée indépendamment de la teneur en dioxyde de carbone, **caractérisé en ce qu'**une valeur prise sur les courbes caractéristiques enregistrées est ajoutée à la valeur de mesure de gaz ou en est soustraite, et que l'influence de l'humidité de l'air sur l'influence du dioxyde de carbone sur la valeur de mesure de gaz soit corrigée, du fait que la valeur de mesure de gaz est corrigée par un facteur à l'aide des courbes caractéristiques enregistrées.

EP 2 336 762 B1

FIG 1

12

EP 2 336 762 B1

## FIG 2

# FIG 3

EP 2 336 762 B1

FIG 4

CPD (ΔΦ) [V]

$CO_2$ [ppm]

T [°C]

r.h. [%]

t [min]

EP 2 336 762 B1

FIG 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 3526348 A1

- EP 1176418 A2

**In der Beschreibung aufgeführte Nicht-Patentliteratur**

- **H.-E. ENDRES et al.** A capacitive CO2 sensor system with suppression of the humidity interference. *Sensors and Actuators B,* 1999, vol. 57, 83-87